# NEUE EUROPÄISCHE PATENTSCHRIFT

(11) **EP 1 720 663 B2**
(45) Veröffentlichungstag und Bekanntmachung des Hinweises auf die Entscheidung über den Einspruch: **15.09.2021**
(45) Hinweis auf die Patenterteilung: 14.11.2018
(21) Anmeldenummer: 05715511.1
(22) Anmeldetag: 24.02.2005
(51) Int. Cl.: B05B 11/00, A61M 11/06, A61M 15/00, A61M 11/00

(54) **ZERSTAUBER MIT KODIERMITTELN**
ATOMISER COMPRISING CODING MEANS
PULVERISATEUR COMPORTANT DES MOYENS DE CODAGE

(30) Priorität: 24.02.2004 DE 102004009434
(43) Veröffentlichungstag der Anmeldung: 15.11.2006
(73) Patentinhaber: Boehringer Ingelheim International GmbH, 55216 Ingelheim am Rhein (DE)
(72) Erfinder: WUTTKE, Gilbert, 44149 Dortmund (DE); GOLBERG, Christian, 45886 Gelsenkirchen (DE); KUNZE, Hubert, 44227 Dortmund (DE); FIOL, Andreas, 22850 Norderstedt (DE); SCHMIEDEL, Guido, 44379 Dortmund (DE); HAUSMANN, Matthias, 44287 Dortmund (DE); SCHYRA, Michael, 42111 Wuppertal (DE); GESER, Johannes, 55218 Ingelheim am Rhein (DE); ZIERENBERG, Bernd, 55411 Bingen (DE)
(74) Vertreter: Von Rohr Patentanwälte Partnerschaft mbB
(86) Internationale Anmeldenummer: PCT/EP2005/001943
(87) Internationale Veröffentlichungsnummer: WO 2005/080002

(56) Entgegenhaltungen:
- WO-A1-99/16487
- US-A- 5 255 823
- US-A- 5 277 334
- US-A- 5 335 823
- US-A- 5 522 378
- US-A- 5 964 416
- US-A1- 2002 079 326
- US-A1- 2003 140 921
- US-B1- 6 257 233

## Beschreibung

Der vorliegende Erfindung betrifft einen Zerstäuber gemäß dem Oberbegriff des Anspruchs 1 sowie eine Verwendung eines Behälters mit einem Zerstäuber gemäß dem Oberbegriff des Anspruchs 17.

Ausgangspunkt der vorliegenden Erfindung ist ein unter dem Handelsnamen "Respimat" von der Boehringer Ingelheim KG angebotener Zerstäuber in Form eines Inhalators, wie im Grundprinzip in der WO 91/14468 A1 und in konkreter Ausgestaltung in der WO 97/12687 A1 (Fig. 6a, 6b) sowie in Fig. 1 und 2 der anliegenden Zeichnung dargestellt, Der Zerstäuber weist als Reservoir für ein zu zerstäubendes Fluid einen einsetzbaren Behälter mit dem Fluid und einen Druckerzeuger mit einer Antriebsfeder zur Förderung und Zerstäubung des Fluids auf.

Zur Vervollständigung der Offenbarung der vorliegenden Patentanmeldung wird vorsorglich auf den kompletten Offenbarungsgehalt sowohl der WO 91/14468 A1 als auch der WO 97/12687 A1 verwiesen, Generell bezieht sich die dortige Offenbarung bevorzugt auf einen Zerstäuber mit einem Federdruck von 5 bis 60 MPa, bevorzugt 10 bis 50 MPa auf das Fluid, mit Volumina pro Hub von 10 bis 50 *µ*l, bevorzugt 10 bis 20 *µ*l, ganz bevorzugt etwa 15 *µ*l pro Hub, Teilchengrößen von bis zu 20 *µ*m, bevorzugt 3 bis 10 *µ*m. Ferner bezieht sich die dortige Offenbarung bevorzugt auf einen Zerstäuber mit zylinderähnlicher Form und einer Größe von etwa 9 cm bis etwa 15 cm in der Länge und etwa 2 cm bis etwa 5 cm in der Breite sowie von einer Düsen-Strahlfächerung von 20° bis 160°, bevorzugt von 80° bis 100°. Derartige Werte gelten auch für den Zerstäuber nach der Lehre der Erfindung als besonders bevorzugte Werte.

Durch Drehen eines Betätigungsteils in Form eines Gehäuseunterteils des Zerstäubers ist die Antriebsfeder spannbar und Fluid in eine Druckkammer des Druckerzeugers saugbar. Nach manueller Betätigung eines Sperrelements wird das Fluid in der Druckkammer von der Antriebsfeder unter Druck gesetzt und zerstäubt, also unter Bildung eines Aerosols ausgegeben. Beim Spannen einerseits und der dann folgenden Zerstäubung andererseits führt der Behälter jeweils eine Hubbewegung aus.

Der Zerstäuber weist eine mechanische Überwachungseinrichtung auf, die zur Zählung von Betätigungen des Zerstäubers das Drehen des Betätigungsteils erfaßt. Der bekannte Zerstäuber arbeitet ausschließlich mechanisch, d, h. ohne Treibgas und ohne Elektrik.

Bei dem bekannten Zerstäuber können Behälter mit unterschiedlichen Fluiden, also insbesondere unterschiedlichen Arzneimitteln, eingesetzt werden. Dies kann zu einer Verwechslungsgefahr bei der Benutzung führen, da der Zerstäuber beispielsweise auf das jeweilige Fluid eingestellt bzw. abgestimmt sein kann und/oder da insbesondere versehentlich ein Behälter mit einem falschen Fluid mit einer falschen Wirkstoffkonzentration oder mit einer falschen Menge eingesetzt werden kann.

Die US 2002/0079326 A1 offenbart eine Vorrichtung zur Erfassung der Betätigung eines Spenders sowie eine Vorrichtung bzw. einen Spender zur Abgabe eines festen oder flüssigen Mediums. Der Spender weist einen Behälter für das Medium und ein daran angeschlossenes Abgabemittel, wie eine Pumpe, auf, Über einen Sensor am Betätigungselement kann erfasst werden, ob eine Schutzkappe für den Spender bzw. das Abgabemittel aufgesetzt ist oder nicht. Eine derartige Konstruktionsweise verhindert jedoch nicht, dass versehentlich ein Behälter mit einem falschen Fluid bzw. einer falschen Wirkstoffkonzentration eingesetzt wird.

Der vorliegenden Erfindung liegt die Aufgabe zugrunde, einen Zerstäuber sowie eine Verwendung eines Behälters mit einem Zerstäuber mit verbesserter Sicherheit gegen Verwechslung des Behälters bei der Benutzung anzugeben.

Die obige Aufgabe wird durch einen Zerstäuber gemäß Anspruch 1 und eine Verwendung eines Behälters mit einen Zerstäuber nach Anspruch 17 gelöst. Vorteilhafte Weiterbildungen des Zerstäubers sind Gegenstand der Unteransprüche.

Eine grundlegende Idee liegt darin, eine Kodierung vorzusehen, so daß nur ein bestimmter Behälter oder mehrere bestimmte Behälter mit einem dafür vorgesehenen Zerstäuber verwendbar, insbesondere in diesen einsetzbar ist bzw. sind. Der Zerstäuber weist hierzu ein erstes Kodiermittel auf. Dem Behälter ist ein zweites Kodiermittel zugeordnet. Die Kodiermittel wirken derart zusammen, daß der Behälter mit dem zugeordneten zweiten Kodiermittel nur dann Zerstäuber einsetzbar oder mit diesem verwendbar ist, wenn die Kodiermittel zueinander passen bzw. eine passende Kodierung bilden.

Die vorschlagsgemäße Lösung führt zu einer wesentlich besseren Sicherheit gegen Verwechslung des Behälters, da ein versehentliches Einsetzen eines falschen Behälters, insbesondere eines Behälters mit einem falschen Fluid, beispielsweise einem falschen Arzneimittel, mit einer falschen Wirkstoffkonzentration oder mit einer falschen Menge, durch die Kodierung ausgeschlossen werden kann. Insbesondere kann so verhindert werden, daß ein für ein bestimmtes Fluid eingestellter Zerstäuber fälschlicherweise für ein anderes Fluid verwendet wird und beispielsweise zu einer überhöhten Dosierung eines Fluids führen kann.

Vorzugsweise arbeitet bzw. wirkt die Kodierung rein mechanisch. Dies gestattet eine sehr einfache und damit kostengünstige Realisierung.

Alternativ oder zusätzlich kann die Kodierung jedoch auch elektrisch, induktiv, kapazitiv, magnetisch und/oder optisch arbeiten bzw. wirken. Insbesondere kann dann aufgrund der Kodierung des Behälters eine Überwachungseinrichtung erfassen, welches Fluid, insbesondere Arzneimittel, eingesetzt worden ist,

Gemäß einem weiteren, auch unabhängig realisierbaren Aspekt weist der Behälter ein Kodiermittel zur eindeutigen Identifizierung des Behälters, des Fluids, der Konzentration eines Wirkstoffs im Fluid und/oder der Fluidmenge auf. Dies ermöglicht eine Identifizierung des tatsächlich mit dem Zerstäuber zum Einsatz kommenden Fluids bzw, Arzneimittels, so daß wiederum die Sicherheit bei der Benutzung wesentlich verbessert werden kann. Dies gilt insbesondere, wenn der Zerstäuber oder beispielsweise eine Überwachungseinrichtung des Zerstäubers das verwendete Fluid mittels des Kodiermittels identifiziert und beispielsweise die Identifizierung speichert, anzeigt oder in sonstiger Weise verarbeitet.

Weitere Vorteile, Merkmale, Eigenschaften und Aspekte der vorliegenden Erfindung ergeben sich aus der folgenden Beschreibung bevorzugter Ausführungsformen anhand der Zeichnung. Es zeigt:
- Fig. 1: einen schematischen Schnitt eines bekannten Zerstäubers im ungespannten Zustand;
- Fig. 2: einen schematischen, um 90° gegenüber Fig. 1 gedrehten Schnitt des bekannten Zerstäubers im gespannten Zustand;
- Fig. 3: eine schematische Schnittdarstellung eines vorschlagsgemäßen Zerstäubers und Behälters gemäß einer ersten Ausführungsform;
- Fig. 4: eine Ansicht des Zerstäubers gemäß Fig. 3 von unten;
- Fig. 5: eine Draufsicht des Behälters gemäß Fig. 3;
- Fig. 6: eine schematische Schnittdarstellung eines vorschlagsgemäßen Zerstäubers und Behälters gemäß einer zweiten Ausführungs-form;
- Fig. 7: eine Schnittansicht gemäß Linie VII-VII von Fig. 6 bei eingesetztem Behälter;
- Fig. 8: eine schematische Schnittdarstellung eines vorschlagsgemäßen Zerstäubers und Behälters gemäß einer dritten Ausführungsform;
- Fig. 9: eine Schnittansicht gemäß Linie IX-IX von Fig. 8 bei eingesetztem Behälter;
- Fig. 10: eine schematische Schnittdarstellung eines vorschlagsgemäßen Zerstäubers und Behälters gemäß einer vierten Ausführungsform;
- Fig. 11: eine Schnittansicht gemäß Linie XI-XI von Fig. 10 bei eingesetztem Behälter;
- Fig. 12: eine schematische Schnittdarstellung eines vorschlagsgemäßen Zerstäubers und Behälters gemäß einer fünften Ausführungs-form;
- Fig. 13: eine Schnittansicht gemäß Linie XIII-XIII von Fig. 12 bei eingesetztem Behälter;
- Fig. 14: eine schematische Schnittdarstellung eines vorschlagsgemäßen Zerstäubers und Behälters gemäß einer sechsten Ausführungs-form;
- Fig. 15: eine Schnittansicht gemäß Linie XV-XV von Fig. 14 bei eingesetztem Behälter;
- Fig. 16: eine schematische Schnittdarstellung eines vorschlagsgemäßen Zerstäubers und Behälters gemäß einer siebten Ausführungs-form; und
- Fig. 17: eine Schnittansicht gemäß Linie XVII-XVII von Fig. 16 bei eingesetztem Behälter.

In den Figuren werden für gleiche oder ähnliche Teile dieselben Bezugszeichen verwendet, wobei entsprechende oder vergleichbare Eigenschaften und Vorteile erreicht werden, auch wenn eine wiederholte Beschreibung weggelassen ist.

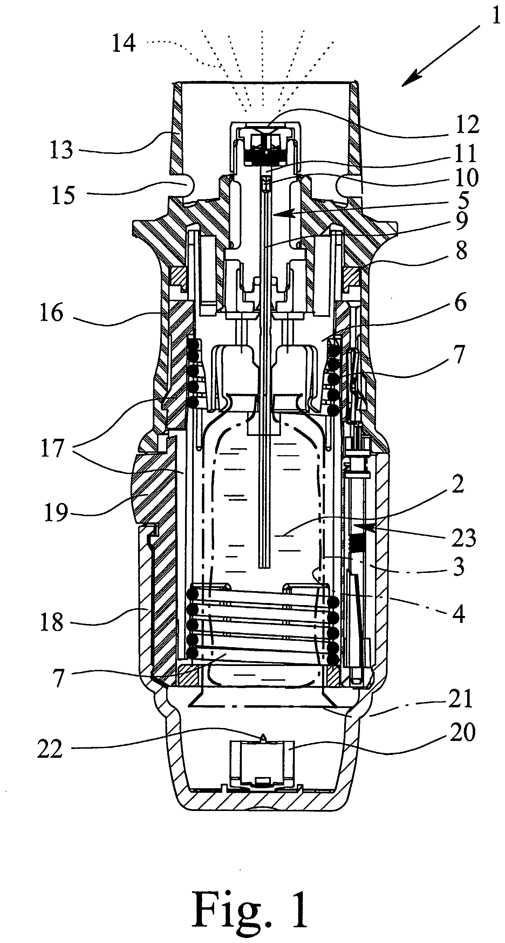

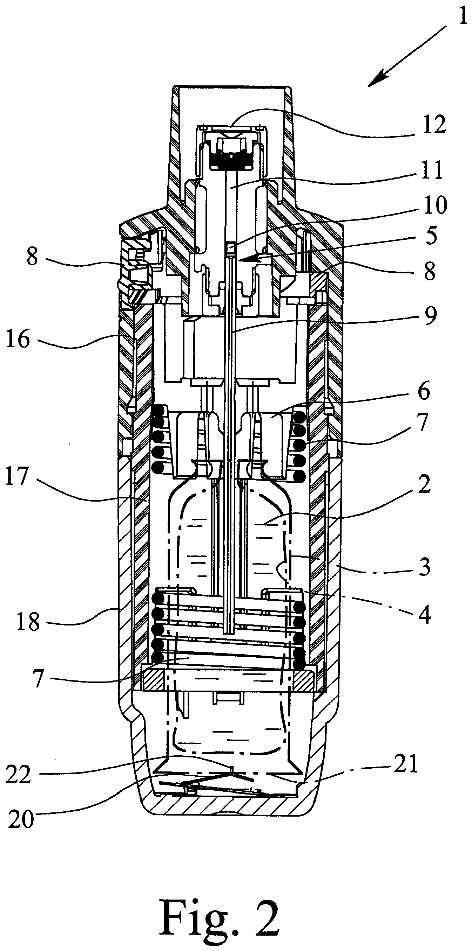

Fig. 1 und 2 zeigen einen bekannten Zerstäuber 1 zur Zerstäubung eines Fluids 2, insbesondere eines hochwirksamen Arzneimittels oder dgl., in einer schematischen Darstellung im ungespannten Zustand (Fig. 1) und gespannten Zustand (Fig. 2). Der Zerstäuber 1 ist insbesondere als tragbarer Inhalator ausgebildet und arbeitet vorzugsweise ohne Treibgas.

Bei Zerstäubung des Fluids 2, vorzugsweise einer Flüssigkeit, insbesondere eines Arzneimittels, wird ein Aerosol gebildet, das von einem nicht dargestellten Benutzer eingeatmet bzw. inhaliert werden kann. Üblicherweise erfolgt das Inhalieren wenigstens einmal täglich, insbesondere mehrmals täglich, vorzugsweise in vorbestimmten Zeitabständen.

Der Zerstäuber 1 weist einen einsetzbaren und vorzugsweise wechselbaren Behälter 3 mit dem Fluid 2 auf, der ein Reservoir für das zu zerstäubende Fluid 2 bildet. Vorzugsweise enthält der Behälter 3 eine ausreichende Menge an Fluid 2 für eine mehrfache Anwendung, insbesondere für eine vorbestimmte Applikationszeit, wie einen Monat, oder für mindestens 50, vorzugsweise mindestens 100, Dosierungen bzw. Zerstäubungen.

Der Behälter 3 ist im wesentlichen zylindrisch bzw. kartuschenartig ausgebildet und von unten, nach Öffnen des Zerstäubers 1, in diesen einsetzbar und ggf. wechselbar. Er ist vorzugsweise starr ausgebildet, insbesondere wobei das Fluid 2 in einem Beutel 4 im Behälter 3 aufgenommen ist.

Der Zerstäuber 1 weist einen Druckerzeuger 5 zur Förderung und Zerstäubung des Fluids 2, insbesondere jeweils in einer vorbestimmten, ggf. einstellbaren Dosiermenge, auf. Der Druckerzeuger 5 weist eine Halterung 6 für den Behälter 3, eine zugeordnete, nur teilweise dargestellte Antriebsfeder 7 mit einem zur Entsperrung manuell betätigbaren Sperrelement 8, ein Förderrohr 9 mit einem Rückschlagventil 10, eine Druckkammer 11 und eine Austragsdüse 12 im Bereich eines Mundstücks 13 auf.

Beim axialen Spannen der Antriebsfeder 7 wird die Halterung 6 mit dem Behälter 3 und dem Förderrohr 9 bei den Darstellungen nach unten bewegt und Fluid 2 aus dem Behälter 3 in die Druckkammer 11 des Druckerzeugers 5 über das Rückschlagventil 10 gesaugt. Da die Austragsdüse 12 einen sehr geringen Strömungsquerschnitt hat und insbesondere als Kapillare ausgebildet ist, ergibt sich eine so starke Drosselwirkung, daß auch ohne Rückschlagventil an dieser Stelle ein Einsaugen von Luft sicher ausgeschlossen ist.

Beim anschließenden Entspannen nach Betätigung des Sperrelements 8 wird das Fluid 2 in der Drucckammer 11 von der das Förderrohr 9 wieder nach oben bewegenden Antriebsfeder 7 - also durch Federkraft - unter Druck gesetzt und über die Austragsdüse 12 ausgegeben, wobei es zerstäubt wird, insbesondere in Partikel im µm- oder nm-Bereich, vorzugsweise lungengängige Partikel mit etwa 5 µm, die eine Wolke bzw. einen Strahl eines Aerosols 14 bilden, wie in Fig. 1 angedeutet. Die Förderung und Zerstäubung des Fluids 2 erfolgen vorzugsweise also rein mechanisch, insbesondere ohne Treibgas und ohne Elektrik.

Ein nicht dargestellter Benutzer kann das Aerosol 14 inhalieren, wobei Zuluft über mindestens eine Zuluftöffnung 15 in das Mundstück 13 saugbar ist.

Der Zerstäuber 1 weist ein Gehäuseoberteil 16 und ein demgegenüber drehbares Innenteil 17 auf, an dem ein insbesondere manuell betätigbares Gehäuseteil 18 vorzugsweise mittels eines Halteelementes 19 lösbar befestigt, insbesondere aufgesteckt, ist. Zum Einsetzen und/oder Auswechseln des Behälters 3 ist das Gehäuseteil 18 vom Zerstäuber 1 lösbar.

Durch manuelles Drehen des Gehäuseteils 18 ist das Innenteil 17 relativ zum Gehäuseoberteil 16 drehbar, wodurch die Antriebsfeder 7 über ein nicht dargestelltes, auf die Halterung 6 wirkendes Getriebe in axialer Richtung spannbar ist. Beim Spannen wird der Behälter 3 axial nach unten bewegt, bis der Behälter 3 eine in Fig. 2 angedeutete Endlage im gespannten Zustand annimmt. Während des Zerstäubungsvorgangs wird der Behälter 3 von der Antriebsfeder 7 wieder in seine Ausgangslage zurückbewegt.

Das Gehäuseteil 18 bildet vorzugsweise ein kappenartiges Gehäuseunterteil und um- bzw. übergreift einen unteren freien Endbereich des Behälters 3. Beim Spannen der Antriebsfeder 7 bewegt sich der Behälter 3 mit seinem Endbereich (weiter) in das Gehäuseteil 18 bzw. zu dessen stirnseitigem Ende hin, wobei eine axial wirkende, im Gehäuseteil 18 angeordnete Feder 20 am Behälterboden 21 zur Anlage kommt und mit einem Anstechelement 22 den Behälter 3 bzw. eine bodenseitige Versiegelung bei der erstmaligen Anlage zur Belüftung ansticht.

Der Zerstäuber 1 weist eine Überwachungseinrichtung 23 auf, die Betätigungen des Zerstäubers 1 zählt, vorzugsweise indem sie ein Drehen des Innenteils 17 zum Gehäuseoberteil 16 erfaßt. Die Überwachungseinrichtung 23 arbeitet beim Darstellungsbeispiel rein mechanisch.

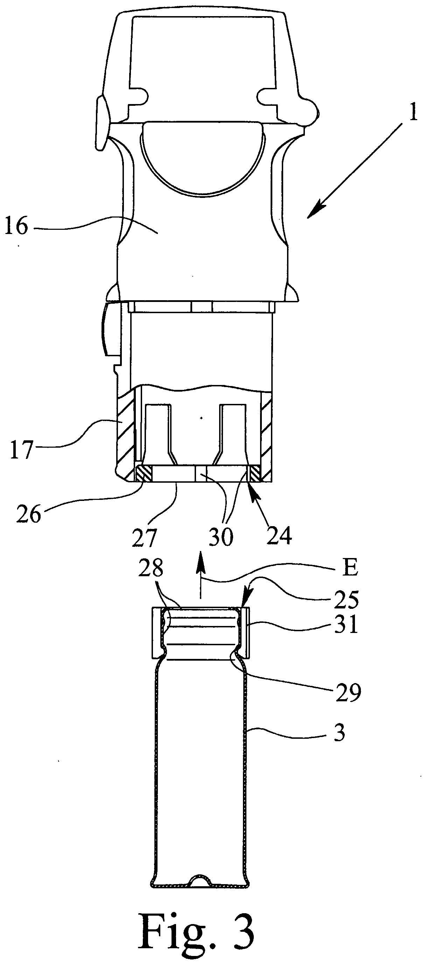

Nachfolgend werden der Aufbau und die Funktionsweise eines vorschlagsgemäßen Zerstäubers 1 und eines vorschlagsgemäßen Behälters 3 näher erläutert, wobei auf die Fig. 3 bis 17 bezug genommen wird, jedoch nur wesentliche Unterschiede gegenüber dem Zerstäuber 1 gemäß Fig. 1 und 2 herausgestellt werden. Die Ausführungen zu Fig. 1 und 2 gelten also entsprechend bzw. ergänzend.

Fig. 3 zeigt in schematischer, ausschnittsweiser Schnittansicht eine erste Ausführungsform des vorschlagsgemäßen Zerstäubers 1 und Behälters 3. Der Zerstäuber 1 weist ein erstes Kodiermittel 24 auf, und dem Behälter 3 ist ein zweites Kodiermittel 25 zugeordnet.

Die Kodiermittel 24, 25 wirken derart zusammen, daß der Behälter 3 mit dem zweiten Kodiermittel 25 nur dann in den Zerstäuber 1 einsetzbar oder mit diesem verwendbar ist, wenn die Kodiermittel 24, 25 eine zueinander passende Kodierung aufweisen. Bei nicht passender Kodierung wird zumindest das vollständige Einsetzen des Behälters 3, insbesondere das Herstellen eines Kontakts des Fluids 2 mit dem Druckerzeuger 5 bzw. Förderrohr 9, verhindert.

Vorzugsweise arbeiten bzw. wirken die Kodiermittel 24, 25 ausschließlich mechanisch.

Alternativ oder zusätzlich können die Kodiermittel 24, 25 auch elektrisch, induktiv, kapazitiv, magnetisch und/oder optisch - also insbesondere berührungslos - zusammenwirken bzw. arbeiten.

Das erste Kodiermittel 24 ist insbesondere nachträglich und/oder unlösbar am Zerstäuber 1 kraft- und/oder formschlüssig angebracht. Beispielsweise kann das erste Kodiermittel 24 an den Zerstäuber 1 angeclipst, angeklemmt, angeklebt, angespritzt, angeschraubt und/oder angeformt sein und/oder in diesen eingebaut sein.

Beim Darstellungsbeispiel ist das erste Kodiermittel 24 an einem Haltering 26 des Zerstäubers 1 angebracht bzw. gebildet. Der Haltering 26 dient einer Widerlagerung der in Fig. 3 nicht dargestellten Antriebsfeder 7 am Innenteil 17 des Zerstäubers 1.

Der Haltering 26 begrenzt bzw. definiert eine Einführöffnung 27 für den Behälter 3. Die Einführöffnung 27 ist am freien Ende des Innenteils 17 gebildet. Durch die Einführöffnung 27 ist der Behälter 3 in den Zerstäuber 1 einführbar, so daß er mit der Halterung 6 des Druckerzeugers 5 in Eingriff bringbar und auch an den Druckerzeuger 5 durch Einführen des Förderrohrs 9 in den Behälter 3 fluidisch anschließbar ist.

Bei der ersten Ausführungsform ist das zweite Kodiermittel 25 am Behälter 3 vorzugsweise unlösbar kraft- und/oder formschlüssig angebracht. Beispielsweise ist das zweite Kodiermittel 25 an den Behälter 3 angeclipst, angeklemmt, angeklebt, angespritzt, angeschraubt und/oder angeformt und/oder in diesen eingebaut.

Beim Darstellungsbeispiel ist das zweite Kodiermittel 25 an einem Kopf 28 des Behälters 3 angeordnet, vorzugsweise wobei das zweite Kodiermittel 25 in eine zum Kopf 28 benachbarte Einziehung bzw. vorzugsweise umlaufende Ringnut 29 des Behälters 3 zur Befestigung am Behälter 3 eingreift.

Das zweite Kodiermittel 25 ist also vorzugsweise formschlüssig am Behälter 3 und insbesondere unlösbar befestigt.

Beim Darstellungsbeispiel ist der Behälter 3 durch eine Linearbewegung in Einsetzrichtung E in den Zerstäuber 1 - durch die Einführöffnung 27 - einsetzbar. Bei passender Kodierung der Kodiermittel 24, 25 sind diese bei der ersten Ausführungsform beim Einsetzen des Behälters 3 aneinander vorbeibewegbar.

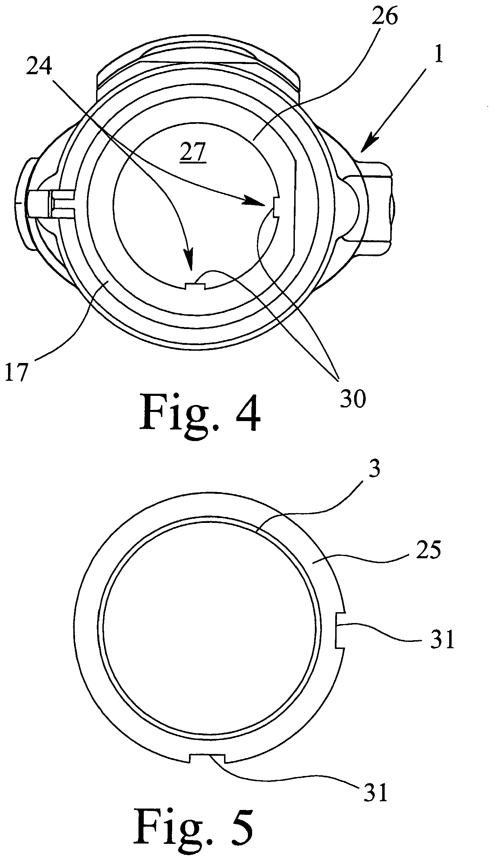

Die bei der ersten Ausführungsform rein mechanisch arbeitenden bzw. wirkenden Kodiermittel 24, 25 weisen vorzugsweise Vorsprünge 30 und/oder Ausnehmungen 31, wie in den Ansichten des Zerstäubers 1 von unten und des Behälters 3 von oben gemäß Fig. 4 bzw. 5 zusätzlich dargestellt, auf, deren Kombination, Anzahl, Form, Größe, Länge, Breite, Tiefe, Kontur und Anordnung - insbesondere ihre Umfangslage - eine Kodierung bilden, die vorzugsweise eindeutig ist. Nur bei passender Kodierung ist der Behälter 3 mit dem zweiten Kodiermittel 25 mit dem ersten Kodiermittel 24 am Zerstäuber 1 in Eingriff bringbar und dementsprechend nur dann in den Zerstäuber 1 einsetzbar bzw. mit diesem verwendbar.

Bei der ersten Ausführungsform weist das erste Kodiermittel 24 zwei vorzugsweise nasenartige, nach innen - also in die Einführöffnung 27 - vorspringende Vorsprünge 30 auf, wie am besten Fig. 4 zu entnehmen ist. Die Vorsprünge 30 springen also quer zur Einsetzrichtung E des Behälters 3 vor.

Die Vorsprünge 30 können bedarfsweise auch gewindeartig oder stegartig ausgebildet sein oder eine sonstige geeignete Form aufweisen.

Bei der ersten Ausführungsform weist das zweite Kodiermittel 25 vorzugsweise zwei Ausnehmungen 31 auf, die quer zur Einsetzrichtung E des Behälters 3 ausgenommen und vorzugsweise nutartig, insbesondere über die gesamte axiale Erstreckung des zweiten Kodiermittels 25 ausgebildet sind.

Beim Darstellungsbeispiel sind die Vorsprünge 30 und die Ausnehmungen 31 derart - insbesondere hinsichtlich ihrer Kombination, Anzahl, Form, Größe und Anordnung - angepaßt, daß der Behälter 3 mit dem zweiten Kodiermittel 25 in den Haltering 26 mit dem ersten Kodiermittel 24 und an diesem vorbei in den Zerstäuber 1 einführbar ist. Die Kodiermittel 24, 25 weisen also zueinander passende Kodierungen auf. Insbesondere arbeiten die Kodiermittel 24, 25 nach dem "Schlüssel-Schloß-Prinzip".

Vorzugsweise erfolgt die Kodierung bei der ersten Ausführungsform durch entsprechende Anordnung der Vorsprünge 30 und Ausnehmungen 31 am Umfang, also durch entsprechende Winkelanordnung. Dies kann auch einfach als Winkelkodierung bezeichnet werden.

Zusätzlich oder alternativ kann auch eine Kodierung über die Anzahl und/oder Form bzw. Größe der Vorsprünge 30 und Ausnehmungen 31 erfolgen.

Bedarfsweise kann das erste Kodiermittel 24 sowohl mindestens einen Vorsprung 30 als auch eine Ausnehmung 31 aufweisen und das zweite Kodiermittel 25 dazu komplementär ausgebildet sein.

Alternativ oder zusätzlich zu der bei der ersten Ausführungsform vorgesehenen Linearbewegung, mit der die Kodiermittel 24, 25 bei passender Kodierung miteinander in Eingriff bringbar sind, kann auch eine Schraub- bzw. Drehbewegung vorgesehen, insbesondere überlagert sein.

Die Vorsprünge 30 und/oder Ausnehmungen 31 können sehr unterschiedliche Formen aufweisen. Entsprechend kann das erste und/oder zweite Kodiermittel 24, 25 insbesondere ringförmig, hülsenförmig, klammerartig, nockenartig, stegartig, nutartig und/oder hakenartig ausgebildet sein.

An Stelle des bei der ersten Ausführungsform vorgesehenen aneinander Vorbeibewegens der Kodiermittel 24, 25 bei passender Kodierung beim Einsetzen des Behälters 3 können die Kodiermittel 24, 25 -je nach Ausbildung und Anordnung - auch bei eingesetztem Behälter 3 in Eingriff bleiben.

Beim Darstellungsbeispiel ist der Behälter 3 vorzugsweise rotationssymmetrisch ausgebildet. Das zweite Kodiermittel 25 ist dementsprechend vorzugsweise nicht rotationssymmetrisch ausgebildet. Entsprechendes gilt dann für das erste Kodiermittel 24, um die passende Kodierung zu ermöglichen.

Das erste und/oder zweite Kodiermittel 24, 25 kann bedarfsweise als separates Teil ausgebildet - wie im Fall des zweiten Kodiermittels 25 am Behälter 3 - oder von einem bestehenden Teil gebildet - wie im Fall des ersten Kodiermittels 24 vom Haltering 26 - sein.

Das zweite Kodiermittel 25 des Behälters 3 stellt vorzugsweise eine eindeutige Identifizierung des Behälters 3, des Fluids 2, der Konzentration eines Wirkstoffs und/oder einer Fluidmenge im Behälter 3 dar. Dies erhöht wesentlich die Sicherheit bei der Benutzung, insbesondere wenn gleichartige oder sogar identische Behälter 3 für verschiedene Fluide 2, insbesondere verschiedene Arzneimittel, für verschiedene Konzentrationen, beispielsweise eines Wirkstoffs, und/oder für verschiedene Fluidmengen eingesetzt werden. Beispielsweise können die Behälter 3 mit Beuteln 4 unterschiedlicher Füllmengen versehen sein. Nachfolgend wird nur auf die Identifizierung des Arzneimittels bzw. Fluids 2 näher eingegangen. Entsprechendes gilt aber auch hinsichtlich der Identifizierung des Behälters 3, der Wirkstoffkonzentration und/oder der Fluidmenge.

In einer einfachsten Ausführungsvariante kann die Identifizierung des Fluids 2 dadurch erfolgen, daß das Kodiermittel 25 für den Benutzer sichtbar das Fluid 2 identifiziert, beispielsweise durch Farbe, Schrift, sonstige Zeichen oder dgl.

Vorzugsweise ist der Zerstäuber 1, insbesondere dessen Überwachungseinrichtung 23, derart ausgebildet, daß eine vorzugsweise selbsttätige Erfassung der Identifikation des Fluids 2 mittels des zweiten Kodiermittels 25 erfolgen kann. Insbesondere ist die Identifikation (beispielsweise ein Name oder sonstige Bezeichnung des Fluids 2 bzw. Arzneimittels) speicherbar, anzeigbar oder in sonstiger Weise verarbeitbar.

Die Identifizierung des Fluids 2 durch das zweite Kodiermittel 25 kann wahlweise durch die passende Kodierung zum ersten Kodiermittel 24, ein sonstiges Erfassen der Kodierung des zweiten Kodiermittels 25 und/oder unabhängig davon durch ein weiteres, geeignetes Identifikationsmittel des Kodiermittels 25 erfolgen.

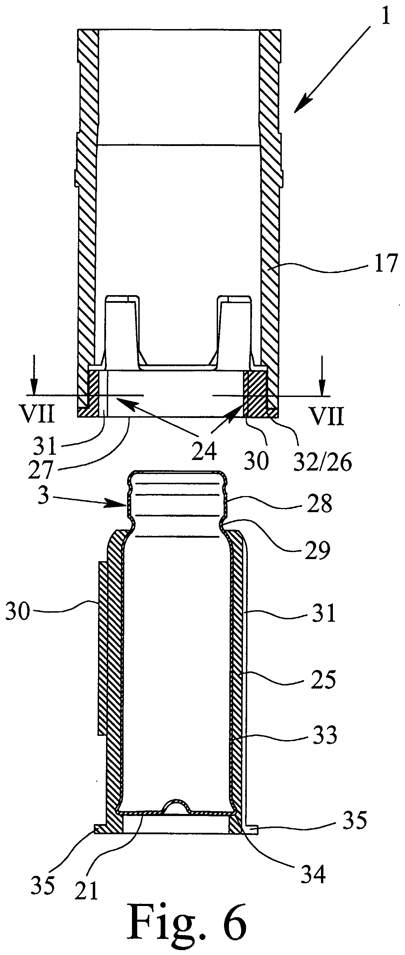

Die schematische, ausschnittsweise Schnittdarstellung gemäß Fig. 6 zeigt eine zweite Ausführungsform des vorschlagsgemäßen Zerstäubers 1 und Behälters 3. Nachfolgend werden für die zweite Ausführungsform und die weiteren Ausführungsformen lediglich wesentliche Unterschiede gegenüber der ersten vorschlagsgemäßen Ausführungsform herausgestellt, wobei sich ansonsten die entsprechenden Eigenschaften und Vorteile ergeben.

Bei der zweiten Ausführungsform ist am Innenteil 17 bzw. an der Einführöffnung 27 ein Einsteckteil 32 mit dem ersten Kodiermittel 24 angeordnet. Das Einsteckteil 32 kann den Haltering 26 bilden oder zusätzlich zu diesem oder einer sonstigen Widerlagerung für die in Fig. 6 nicht gezeigte Antriebsfeder 7 vorgesehen sein.

Vorzugsweise ist das Einsteckteil 32 erst nachträglich am Zerstäuber 1 anbringbar, um eine bedarfgerechte Konfigurierung bzw. Kodierung des Zerstäubers 1 zu ermöglichen. Insbesondere kann die Anbringung des Einsteckteils 32 bedarfsweise beim Endhersteller oder beispielsweise erst bei einem Apotheker oder von einem Arzt erfolgen.

Das Einsteckteil 32 ist vorzugsweise rastend oder in sonstiger Weise am Zerstäuber 1, insbesondere am Innenteil 17, anbringbar. Nach der Abringung ist das Einsteckteil 32 vorzugsweise nicht mehr lösbar.

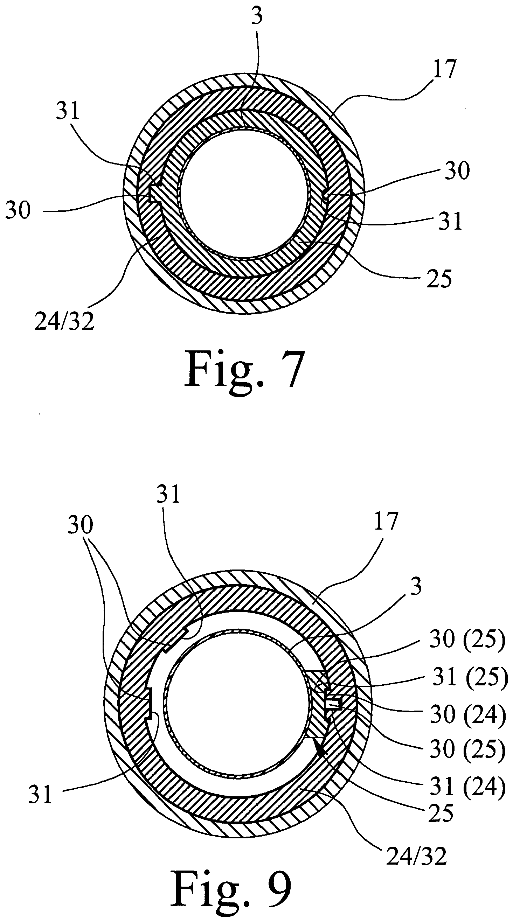

Bei der zweiten Ausführungsform weist das erste Kodiermittel 24 eine im Radialschnitt eckige bzw. nutartige Ausnehmung 31 und einen im Radialschnitt gerundeten bzw. nasenartigen Vorsprung 30 auf, wie der Schnittdarstellung bei eingesetztem Behälter 3 gemäß Fig. 7 zu entnehmen ist.

Bei der zweiten Ausführungsform ist das zweite Kodiermittel 25 vorzugsweise hülsenartig ausgebildet und an einem insbesondere zylindrischen Mantel 33 des Behälters 3 angebracht. Insbesondere umgibt das zweite Kodiermittel 25 den Mantel 33 zumindest im wesentlichen vollständig peripher und/oder über die gesamte axiale Länge, wie in Fig. 6 angedeutet.

Bei der zweiten Ausführungsform greift das zweite Kodiermittel 25 in die radiale Einziehung bzw. Ringnut 29 im Bereich des Kopfs 28 des Behälters 3 zur axialen Festlegung ein. Weiter erstreckt sich das zweite Kodiermittel 25 in diesem Fall bis über den Behälterboden 21 hinaus und umfaßt bzw. umgreift hierbei einen radial verbreiterten Endbereich bzw. Bodenrand 34 des Behälters 3.

Insbesondere erstreckt sich das zweite Kodiermittel 25 über den Behälterboden 21 hinaus und weist im Bereich diesen Endes eine Ringschulter 35 oder dgl. auf, die radial nach außen vorsteht und einen Einführanschlag beim Einführen des Behälters 3 in den Zerstäuber 1 im eingesetzten Zustand und/oder eine mögliche Handhabe für einen Benutzer zum Entfernen bzw. Herausnehmen des Behälters 3 aus den Zerstäuber 1 bildet.

Bei der zweiten Ausführungsform weist das zweite Kodiermittel 25 eine nutartige bzw. rillenartige Ausnehmung 31 auf, die sich vorzugsweise zumindest im wesentlichen über die gesamte axiale Länge des zweiten Kodiermittels 25 und insbesondere des Behälters 3 erstreckt.

Weiter weist das zweite Kodiermittel 25 einen vorzugsweise stegartigen Vorsprung 30 auf, der ebenfalls in Axialrichtung auf der Mantelfläche des zweiten Kodiermittels 25 verläuft. Im Gegensatz zur Ausnehmung 31 erstreckt sich der Vorsprung 30 beim Darstellungsbeispiel nicht über die gesamte axiale Länge des zweiten Kodiermittels 25, sondern nur über einen gewissen Teil.

Im Gegensatz zur ersten Ausführungsform bleiben bei der zweiten Ausführungsform das erste und zweite Kodiermittel 24, 25 - zumindest der in Fig. 6 und 7 rechte Vorsprung 30 und die rechte Ausnehmung 31 - auch bei vollständig eingesetztem Behälter 3 in Eingriff, insbesondere längsverschiebbar und drehgesichert.

Wie bei der ersten Ausführungsform sind auch bei der zweiten Ausführungsform die Kodiermittel 24, 25 axial ineinander schiebbar.

Bei der zweiten Ausführungsform kann zusätzlich oder alternativ zu der bei der ersten Ausführungsform angesprochenen Winkelkodierung auch eine Längenkodierung vorgesehen sein. Beispielsweise kann die axiale Lage oder Länge des Vorsprungs 30 des zweiten Kodiermittels 25 und die axiale Länge der dazu korrespondierenden Ausnehmung 31 des ersten Kodiermittels 24 je nach gewünschter Kodierung variieren, insbesondere so daß bei nicht passender Kodierung der Behälter 3 mit dem zweiten Kodiermittel 25 zumindest nicht vollständig in den Zerstäuber 1 bzw. die Einführöffnung 27 bzw. das erste Kodiermittel 24 einführbar oder einsetzbar, insbesondere einschiebbar ist.

Es ist anzumerken, daß der Behälter 3 und/oder das zweite Kodiermittel 25 auch bei vollständig eingesetztem Behälter 3 mit dem freien Ende noch aus dem Zerstäuber 1, insbesondere dem Innenteil 17 bzw. der Einführöffnung 27, vorragen kann bzw. können, und zwar beim Darstellungsbeispiel in das in den Fig. 3 bis 11 nicht dargestellte, lösbare Gehäuse(unter)teil 18 des Zerstäubers 1.

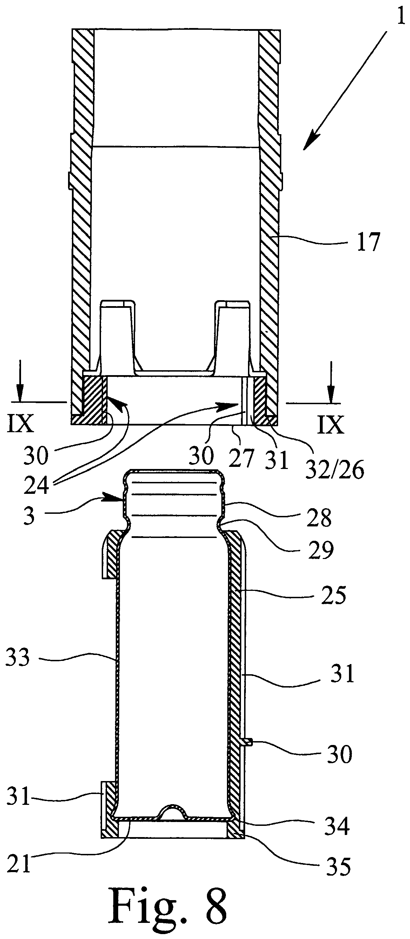

Bei einer dritten, in Fig. 8 und 9 dargestellten Ausführungsform ist das zweite Kodiermittel 25 am Behälter 3 im Gegensatz zur zweiten Ausführungsform in einem Mantelbereich durchbrochen bzw. unterbrochen. Diese Aussparung bzw. Durchbrechung gestattet beispielsweise die Anbringung einer Beschriftung, insbesondere eines Etiketts oder dgl., unmittelbar auf dem Mantel 33 des Behälters 3, so daß das zweite Kodiermittel 25 unabhängig von dieser Beschriftung am Behälter 3 anbringbar ist und diese Beschriftung sichtbar bleibt.

Die dritte Ausführungsform weist im Gegensatz zur zweiten Ausführungsform eine etwas andere Konfiguration und Anordnung der Vorsprünge 30 und Ausnehmungen 31 der Kodiermittel 24, 25 auf, wie der Schnittdarstellung bei eingesetztem Behälter 3 gemäß Fig. 9 zu entnehmen ist. Die Klammern geben jeweils an, welchem Kodiermittel 24 oder 25 die Vorsprünge 30 und Ausnehmungen 31 zuzuordnen sind.

Insbesondere weist das zweite Kodiermittel 25 - bei der Darstellung gemäß Fig. 8 und 9 auf der rechten Seite - eine zumindest im wesentlichen durchgehende Axialnut als Ausnehmung 31 und einen nasenförmigen Vorsprung 30 in dieser Axialnut an einer bestimmten Axiallage auf. Das erste Kodiermittel 24 ist dementsprechend mit einer dazu korrespondierenden Ausnehmung 31 in einem Vorsprung 30 versehen.

Bei der dritten Ausführungsform ist insbesondere sowohl eine Winkelkodierung als auch eine Längenkodierung im genannten Sinne vorgesehen.

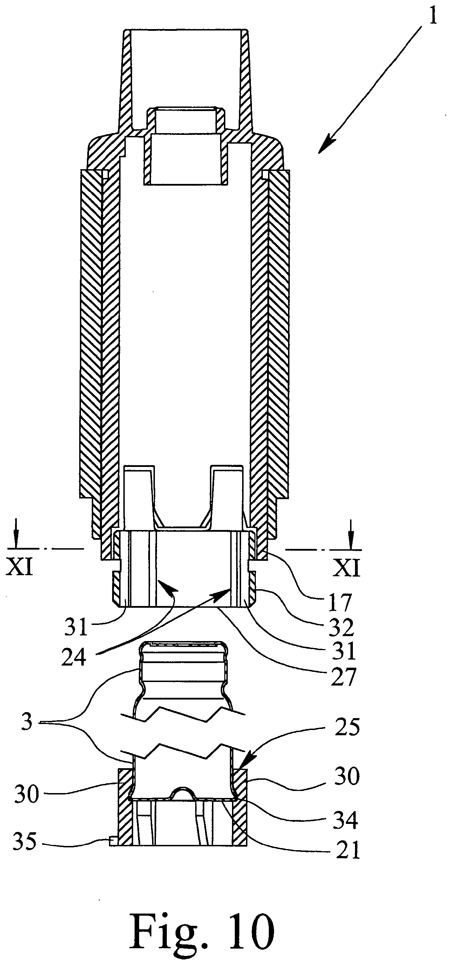

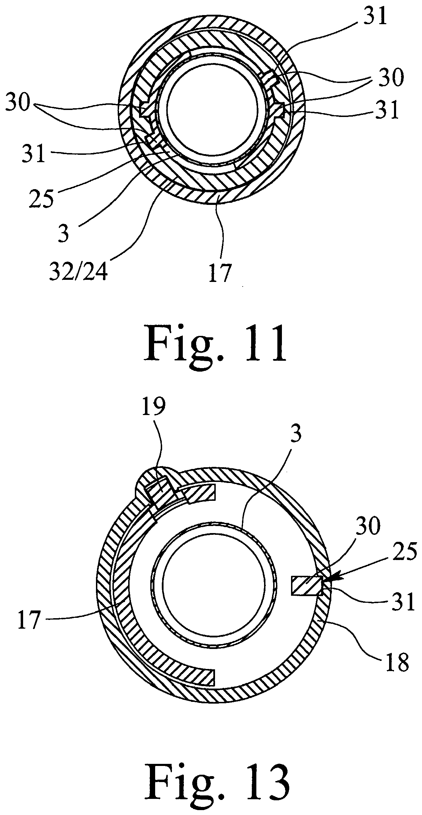

Bei einer in Fig. 10 und 11 dargestellten, vierten Ausführungsform ist das zweite Kodiermittel 25 vorzugsweise im wesentlichen ringförmig ausgebildet und/oder am freien Ende des Behälters 3 bzw. im Bereich des Behälterbodens 21 angeordnet. Vorzugsweise umgreift das zweite Kodiermittel 25 wiederum den insbesondere verbreiterten Endbereich bzw. Bodenrand 34 des Behälters 3 und ist dadurch zumindest in axialer Richtung formschlüssig am Behälter 3 befestigt.

Die Schnittdarstellung bei eingesetztem Behälter 3 gemäß Fig. 11 veranschaulicht die bei der vierten Ausführungsform vorgesehene Konfiguration und Anordnung der Vorsprünge 30 und Ausnehmungen 31.

Bei der ersten bis vierten Ausführungsform sind die Vorsprünge 30 und Ausnehmungen 31 des ersten Kodiermittels 24 vorzugsweise jeweils an einer radialen Innenfläche angeordnet bzw. radial innenwirkend ausgebildet und bei dem zweiten Kodiermittel 25 dann dementsprechend an einer radialen Außenfläche bzw. radial nach außen wirkend. Dies ist jedoch nicht unbedingt erforderlich. Insbesondere kann der Wirkungsmechanismus bzw. die Kodierung auch umgekehrt sein und/oder in axialer Richtung bzw. stirnseitig wirken.

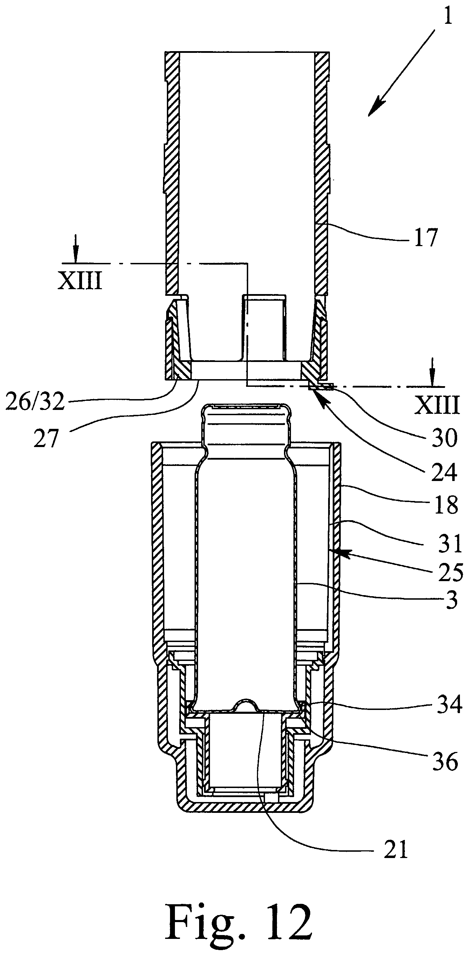

Fig. 12 und 13 zeigen eine fünfte Ausführungsform des vorschlagsgemäßen Zerstäubers 1 und Behälters 3.

Bei der fünften Ausführungsform ist das erste Kodiermittel 24 wiederum als Einsteckteil 32 ausgebildet oder daran angeordnet, wobei das Einsteckteil 32 vorzugsweise rastend und/oder unlösbar am Innenteil 17 anbringbar ist.

Das erste Kodiermittel 24 weist einen radial nach außen ragenden, insbesondere das Innenteil 17 radial nach außen überragenden Vorsprung 30 auf.

Der Behälter 3 ist vorzugsweise unlösbar mit dem Gehäuseteil 18 verbunden, insbesondere mittels eines Verbindungselements 36, das den verbreiterten Endbereich bzw. Bodenrand 34 des Behälters 3 formschlüssig umgreift.

Bei der fünften Ausführungsform ist im Gegensatz zur ersten bis vierten Ausführungsform das zweite Kodiermittel 25 vorzugsweise an einem mit dem Zerstäuber 1 verbindbaren Gehäuseteil, hier dem Gehäuse(unter)teil 18 des Zerstäubers 1 angeordnet oder davon gebildet.

Insbesondere weist das zweite Kodiermittel 25 bei der fünften Ausführungsform eine radial nach innen geöffnete Ausnehmung 31 auf, die axial verläuft und vorzugsweise auf der Innenseite der Außenwandung des Gehäuseteils 18 gebildet ist.

Bei passender Kodierung der Kodiermittel 24, 25 kann beim Zusammenbau des Zerstäubers 1 - also beim Aufsetzen des Gehäuseunterteils 18 mit dem Behälter 3 - der Vorsprung 30 des ersten Kodiermittels 24 in die Ausnehmung 31 des zweiten Kodiermittels 25 eingreifen bzw. eingeschoben werden, wie in Fig. 13 bei eingesetztem Behälter 3 bzw. montiertem Gehäuseteil 18 angedeutet. Bei nicht passender Kodierung ist hingegen das Anbringen des Gehäuseteils 18 am Zerstäuber 1 -insbesondere das Aufschieben auf das Innenteil 17 - blockiert.

Statt der bevorzugten unlösbaren Verbindung des Gehäuse(unter)teils 18 mit dem Behälter 3 kann auch zwischen diesen eine Kodierung im genannten Sinne vorgesehen sein, so daß der Behälter 3 nur bei passender Kodierung in das Gehäuse(unter)teil 18 einsetzbar ist.

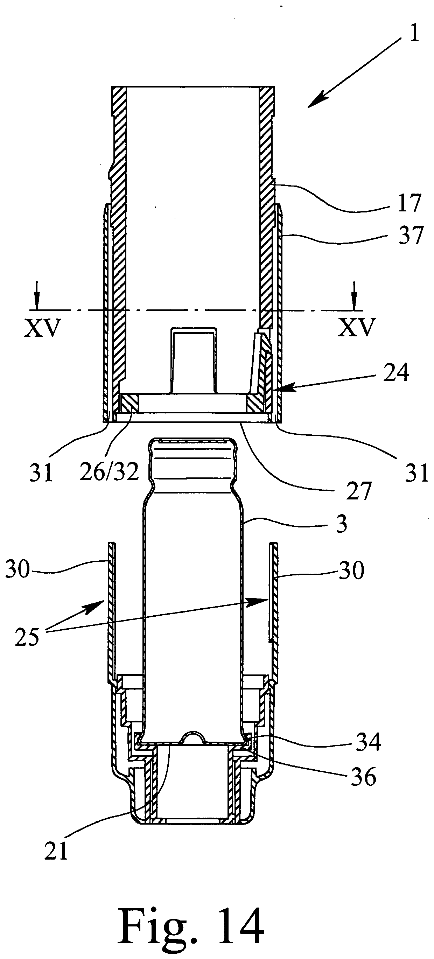

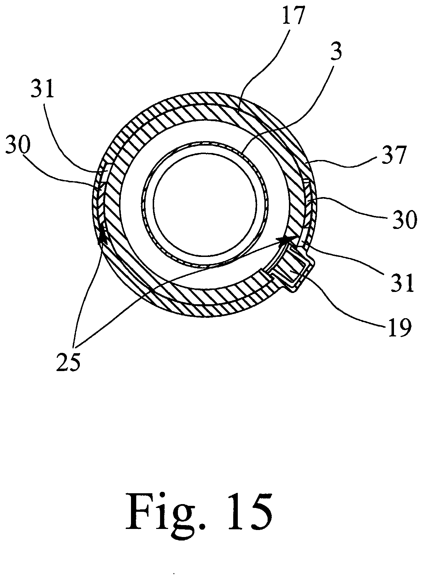

Fig. 14 und 15 zeigen eine sechste Ausführungsform des vorschlagsgemäßen Zerstäubers 1 und Behälters 3, wobei Fig. 14 das Gehäuseteil 18 nicht zeigt und Fig. 15 den eingesetzten bzw. zusammengebauten Zustand zeigt.

Im Gegensatz zur fünften Ausführungsform ist das Gehäuseteil 18 bajonettartig - insbesondere also mit einer kombinierten und/oder überlagerten Linear- und Drehbewegung - am Zerstäuber 1 bzw. dessen Innenteil 17 anbringbar bzw. befestigbar. Hierzu weist das erste Kodiermittel 24 vorzugsweise eine am Zerstäuber 1 bzw. dessen Innenteil 17 anbringbare bzw. angebrachte Verbindungshülse 37 auf. Das zweite Kodiermittel 25 weist armartige, am freien Ende vorzugsweise hakenartige Vorsprünge 30 auf, die die genannte bajonettartige Anbringung des Gehäuseteils 18 am Zerstäuber 1 bei passender Kodierung durch Eingriff in die entsprechend gestalteten Ausnehmungen 31 gestatten.

Die Ausnehmungen 31 des ersten Kodiermittels 24 - insbesondere an der Verbindungshülse 37 - sind bei der sechsten Ausführungsform vorzugsweise im wesentlichen schlitzartig ausgebildet und vorzugsweise derart hinterschnitten, daß im montierten Zustand ein axiales Abziehen des Gehäuseteils 18 durch Formschluß mit den armartigen Vorsprüngen 30 des zweiten Kodiermittels 25 nicht möglich ist.

Beim Darstellungsbeispiel greifen die Vorsprünge 30 des zweiten Kodiermittels 25 derart in die Ausnehmungen 31, vorzugsweise im Bereich der Innenseite der Verbindungshülse 37, ein, so daß die Verbindungshülse 37 bei der Anbringung des Gehäuseteils 18 am Zerstäuber 1 in den Zwischenraum bzw. Ringraum zwischen den Vorsprüngen 30 des zweiten Kodiermittels 25 und der Innenwandung des vorzugsweise zumindest im wesentlichen zylindrischen Abschnitts des Gehäuseteils 18 eingreift bzw. eingeschoben wird.

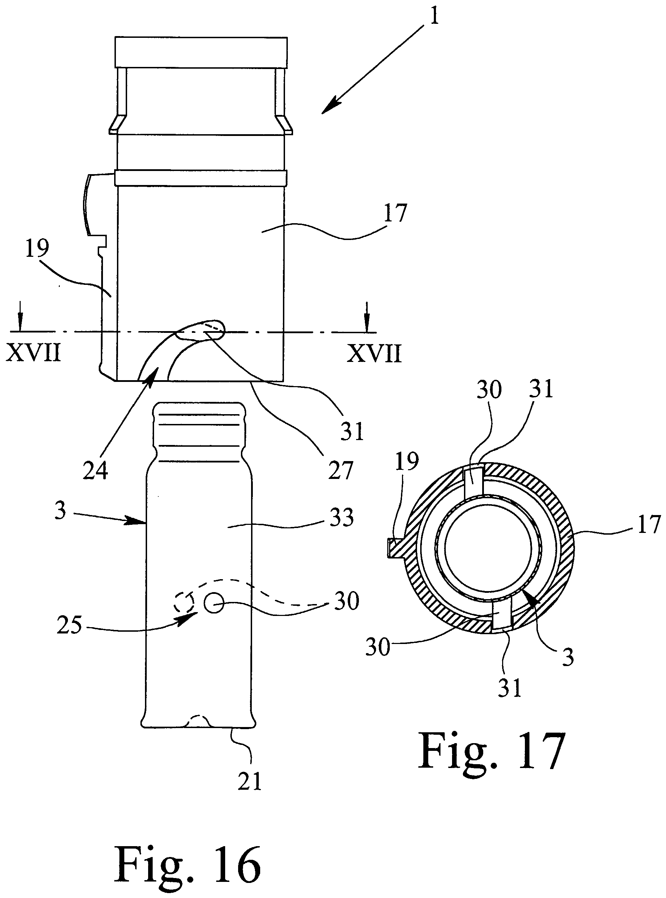

Fig. 16 und 17 zeigen eine siebte Ausführungsform des vorschlagsgemäßen Zerstäubers 1 und Behälters 3. Die siebte Ausführungsform ähnelt der sechsten Ausführungsform. Zur Anbringung des Gehäuseteils 18 ist hier insbesondere eine schraubenartige Bewegung des Gehäuseteils 18 mit dem Behälter 3 relativ zum Zerstäuber 1, insbesondere dessen Innenteil 17, vorgesehen.

Der Zerstäuber 1 bzw. das Innenteil 17 weist das erste Kodiermittel 24, und zwar schlitzartige, vorzugsweise im wesentlichen schräg und gebogen in bzw. an dem Innenteil 17 verlaufende Ausnehmungen 31 auf.

Das zweite, am Gehäuseteil 18 oder unmittelbar am Behälter 3 angeordnete Kodiermittel 25 weist bei der siebten Ausführungsform vorzugsweise knopfartige, an die Ausnehmungen 31 des ersten Kodiermittels 24 angepaßte Vorsprünge 30 auf, so daß bei passender Kodierung der Kodiermittel 24, 25 das Gehäuseteil 18 zusammen mit dem Behälter 3 mit einer schraubenartigen Bewegung am Zerstäuber 1 anbringbar ist, wie in Fig. 17 ohne Gehäuseteil 18 dargestellt.

An Stelle oder zusätzlich zu einer knopfartigen Ausbildung an der Mantelfläche 33 können die Vorsprünge 30 auch angepaßt - fingerartig oder -armartig ausgeführt sein, wenn die Ausnehmungen 31 entsprechend verlaufen, und damit in die Ausnehmungen 31 bei passender Kodierung eingreifen.

Ein besonderer Vorteil der vorliegenden Erfindung liegt darin, daß das erste und/oder zweite Kodiermittel 24, 25 nachträglich anbringbar ist bzw. sind, so daß beispielsweise der Zerstäuber 1 und/oder der Behälter 3 im Herstellprozeß möglichst spät kodiert wird bzw. werden und daß die Kodierung frei gewählt und damit endgültig festgelegt wird. Alternativ oder zusätzlich ist die Kodierung zunächst einstellbar und dann endgültig festlegbar.

Gemäß einer nicht dargestellten Variante können die Kodiermittel 24, 25 derart ausgebildet sein, daß erst mit dem erstmaligen Einsetzen des Behälters 3 in den Zerstäuber 1 zumindest die Kodierung des ersten Kodiermittels 24 zwangsweise festgelegt wird, beispielsweise durch irreversible Verformung oder Abbrechen von Eingriffselementen oder dgl.

Bedarfsweise können die Kodiermittel 24, 25 jeweils Vorsprünge 30, Ausnehmungen 31 oder dgl. aufweisen, die in unterschiedlichen Ebenen und/oder axialen Lagen in Einführrichtung angeordnet sind.

Die voranstehend beschriebenen Ausführungsformen - insbesondere einzelne Elemente und Aspekte der Ausführungsformen - können je nach Bedarf miteinander kombiniert und/oder kinematisch umgekehrt werden. Insbesondere können die Anzahl und Anordnung der Vorsprünge 30 und Ausnehmungen 31 bedarfsgerecht variiert und an die jeweiligen Gegebenheiten angepaßt werden.

### Bezugszeichenliste

- 1: Zerstäuber
- 2: Fluid
- 3: Behälter
- 4: Beutel
- 5: Druckerzeuger
- 6: Halterung
- 7: Antriebsfeder
- 8: Sperrelement
- 9: Förderrohr
- 10: Rückschlagventil
- 11: Druckkammer
- 12: Austragsdüse
- 13: Mundstück
- 14: Aerosol
- 15: Zuluftöffnung
- 16: Gehäuseoberteil
- 17: Innenteil
- 18: Gehäuseteil (Unterteil)
- 19: Halteelement
- 20: Feder (im Gehäuseunterteil)
- 21: Behälterboden
- 22: Anstechelement
- 23: Überwachungseinrichtung
- 24: erstes Kodiermittel
- 25: zweites Kodiermittel
- 26: Haltering
- 27: Einführöffnung (Zerstäuber)
- 28: Kopf (Behälter)
- 29: Einziehung
- 30: Vorsprünge
- 31: Ausnehmungen
- 32: Einsteckteil
- 33: Mantel (Behälter)
- 34: Endbereich
- 35: Ringschulter
- 36: Verbindungselement
- 37: Verbindungshülse

## Patentansprüche

1. Zerstäuber (1) für ein Fluid (2), mit einem das Fluid (2) enthaltenden, einsetzbarem Behälter (3), mit einem nur mechanisch arbeitenden und manuell betätigbaren Druckerzeuger (5) zur Förderung und/oder Zerstäubung des Fluids (2) und vorzugsweise mit einem zum Einsetzen des Behälters (3) lösbaren Gehäuseteil (18), wobei der Zerstäuber (1) ein erstes Kodiermittel (24) aufweist und dem Behälter (3) ein zweites Kodiermittel (25) zugeordnet ist, wobei die Kodiermittel (24, 25) derart zusammenwirken, dass der Behälter (3) mit dem zugeordneten zweiten Kodiermittel (25) nur dann in den Zerstäuber (1) einsetzbar oder mit diesem verwendbar ist, wenn die Kodiermittel (24, 25) eine zueinander passende Kodierung aufweisen, wobei mindestens eines der Kodiermittel (24, 25) mindestens einen quer zur Einsetzrichtung (E) des Behälters (3) vorspringenden, insbesondere stegartigen, nasenartigen oder gewindeartigen Vorsprung (30) aufweist und wobei mindestens eines der Kodiermittel (24, 25) mindestens eine quer zur Einsetzrichtung (E) des Behälters (3) ausgenommene, vorzugsweise nutartige oder gewindeartige Ausnehmung (31) aufweist,
**dadurch gekennzeichnet,**
**dass** der Vorsprung (30) und die Ausnehmung (31) derart ausgebildet sind und zusammenwirken, dass sie bei passender Kodierung beim Einsetzen des Behälters (3) in den Zerstäuber (1) in Eingriff bringbar und axial ineinander schiebbar sind.

2. Zerstäuber nach Anspruch 1, **dadurch gekennzeichnet, dass** die Kodiermittel (24, 25) derart ausgebildet sind, dass bei nicht passender Kodierung zumindest das vollständige Einsetzen des Behälters (3), insbesondere das Herstellen eines Kontakts des Fluids (2) mit dem Druckerzeuger (5) oder einem Förderrohr (9), verhindert ist, und/oder dass die Kodiermittel (24, 25) derart ausgebildet sind, dass sie bei nicht passender Kodierung das Anbringen des Gehäuseteils (18) am Zerstäuber (1) verhindern.

3. Zerstäuber nach einem der voranstehenden Ansprüche, **dadurch gekennzeichnet, dass** die Kodiermittel (24, 25), vorzugsweise ausschließlich, mechanisch zusammenwirken, und/oder dass die Kodiermittel (24, 25) elektrisch, induktiv, kapazitiv, magnetisch und/oder optisch zusammenwirken, und/oder dass das erste Kodiermittel (24) unlösbar am Zerstäuber (1) anbringbar bzw. angebracht ist.

4. Zerstäuber nach einem der voranstehenden Ansprüche, **dadurch gekennzeichnet, dass** das erste Kodiermittel (24) an dem Zerstäuber (1) kraft- und/oder formschlüssig angebracht, insbesondere angeclipst, angeklemmt, angeklebt, angespritzt, angeschraubt und/oder angeformt ist und/oder in diesen eingebaut ist, und/oder dass das erste Kodiermittel (24) an oder im Bereich einer Einführöffnung (27) des Zerstäubers (1) für den Behälter (3) angeordnet ist, und/oder dass das erste Kodiermittel (24) an oder im Bereich einer Halterung (6) des Zerstäubers (1) für den Behälter (3) angeordnet ist.

5. Zerstäuber nach einem der voranstehenden Ansprüche, **dadurch gekennzeichnet, dass** das zweite Kodiermittel (25) zu dem Fluid (2) im Behälter (3), einer Wirkstoffkonzentration des Fluids (2) und/oder einer Fluidmenge korrespondiert.

6. Zerstäuber nach einem der voranstehenden Ansprüche, **dadurch gekennzeichnet, dass** das zweite Kodiermittel (25) am Behälter (3) vorzugsweise unlösbar anbringbar bzw. angebracht ist, insbesondere wobei das zweite Kodiermittel (25) an dem Behälter (3) kraft- und/oder formschlüssig angebracht, insbesondere angeclipst, angeklemmt, angeklebt, angespritzt, angeschraubt und/oder angeformt ist und/oder in diesen eingebaut ist.

7. Zerstäuber nach einem der voranstehenden Ansprüche, **dadurch gekennzeichnet, dass** das zweite Kodiermittel (25) an einem Kopf (28) des Behälters (3) angeordnet ist, insbesondere wobei der Behälter (3) benachbart zum Kopf (28) eine radiale Einziehung (29) aufweist, in die das zweite Kodiermittel (25) zur Befestigung am Behälter (3) eingreift, und/oder dass das zweite Kodiermittel (25) an einem vorzugsweise zylindrischen Mantel (33) des Behälters (3) angebracht ist.

8. Zerstäuber nach einem der voranstehenden Ansprüche, **dadurch gekennzeichnet, dass** das zweite Kodiermittel (25) an einem Boden (21) des Behälters (3) angebracht ist, insbesondere wobei der Behälter (3) einen vorzugsweise radial verbreiterten Endbereich (34) aufweist, den das zweite Kodiermittel (25) zur Befestigung am Behälter (3) umgreift.

9. Zerstäuber nach einem der Ansprüche 1 bis 5, **dadurch gekennzeichnet, dass** das zweite Kodiermittel (25) am Gehäuseteil (18) angeordnet, unlösbar angebracht oder davon gebildet ist.

10. Zerstäuber nach Anspruch 9, **dadurch gekennzeichnet, dass** das zweite Kodiermittel (25) eine radial nach innen geöffnete Ausnehmung (31) aufweist, die axial verläuft und vorzugsweise auf der Innenseite der Außenwandung des Gehäuseteils (18) gebildet ist.

11. Zerstäuber nach Anspruch 9 oder 10, **dadurch gekennzeichnet, dass** das erste Kodiermittel (24) als Einsteckteil (32) ausgebildet ist, das rastend und/oder unlösbar am Innenteil (17) anbringbar ist.

12. Zerstäuber nach einem der voranstehenden Ansprüche, **dadurch gekennzeichnet, dass** der Behälter (3) unlösbar mit dem Gehäuseteil (18) verbunden ist.

13. Zerstäuber nach einem der voranstehenden Ansprüche, **dadurch gekennzeichnet, dass** mindestens eines der Kodiermittel (24, 25) ringförmig, hülsenförmig, klammerartig, nockenartig, stegartig, nutartig und/oder hakenartig ausgebildet ist.

14. Zerstäuber nach einem der voranstehenden Ansprüche, **dadurch gekennzeichnet, dass** die Kodiermittel (24, 25) bei passender Kodierung durch eine Linear- und/oder Schraubbewegung miteinander in Eingriff bringbar sind, und/oder dass die Kodiermittel (24, 25) bei passender Kodierung beim Einsetzen des Behälters (3) aneinander vorbei bewegbar sind oder bei eingesetztem Behälter (3) miteinander in Eingriff stehen oder die Kodierung des ersten und/oder zweiten Kodiermittels (24, 25), vorzugsweise nachträglich, einstellbar und/oder endgültig festlegbar ist.

15. Zerstäuber nach einem der voranstehenden Ansprüche, **dadurch gekennzeichnet, dass** die Kodiermittel (24, 25), insbesondere Vorsprünge (30) und/oder Ausnehmungen (31) der Kodiermittel (24, 25), nur bei passender Kodierung in ihrer Winkelanordnung bezüglich der Einsetzrichtung (E) des Behälters (3) korrespondieren, und/oder dass die Kodiermitel (24, 25), insbesondere Vorsprünge (30) und/oder Ausnehmungen (31) der Kodiermittel (24, 25), nur bei passender Kodierung in ihrer Längserstreckung in Einsetzrichtung (E) des Behälters (3) korrespondieren, und/oder dass das erste Kodiermittel (24) und das zweite Kodiermittel (25) bei passender Kodierung komplementär zueinander ausgebildet sind.

16. Zerstäuber nach einem der voranstehenden Ansprüche, **dadurch gekennzeichnet, dass** der Behälter (3) rotationssymmetrisch und/oder starr ausgebildet ist, und/oder dass der Druckerzeuger (5) gegen Federkraft spannbar ist, und/oder dass die Druckerzeugung bzw. Zerstäubung rein mechanisch, insbesondere treibgasfrei, vorzugsweise durch Federkraft, erfolgt, und/oder dass der Zerstäuber (1) als Inhalator, insbesondere zur medizinischen Aerosol-Therapie, ausgebildet ist.

17. Verwendung eines Behälters (3) mit einem Zerstäuber (1), wobei der Zerstäuber (1) einen Druckerzeuger (5) zur Förderung und/oder Zerstäubung eines Fluids (2) und vorzugsweise einen zum Einsetzen des Behälters (3) lösbaren Gehäuseteil (18) und ein erstes Kodiermittel (24) aufweist, wobei dem Behälter (3) ein zweites Kodiermittel (25) zugeordnet ist und die Kodiermittel (24, 25) derart zusammenwirken, dass der Behälter (3) mit dem zugeordneten zweiten Kodiermittel (25) nur dann in den Zerstäuber (1) einsetzbar ist, wenn die Kodiermittel (24, 25) eine zueinander passende Kodierung aufweisen,
**dadurch gekennzeichnet,**
**dass** die Kodierung zunächst eigestellt und dann endgültig festgelegt ist, wobei die Kodiermittel (24, 25) derart ausgebildet sind, dass erst mit dem erstmaligen Einsetzen des Behälters (3) in den Zerstäuber (1) zumindest die Kodierung des ersten Kodiermittels (24) zwangsweise festgelegt wird.

## Claims

1. Nebulizer (1) for a fluid (2), with an insertable container (3) containing the fluid (2), with a only mechanically operating and manually actuatable pressure generator (5) for conveying and/or nebulizing the fluid (2) and preferably with a housing part (18) which is detachable for the insertion of the container (3), wherein the nebulizer (1) comprises a first coding means (24) and the container (3) is associated with a second coding means (25), the coding means (24, 25) cooperating with one another such that the container (3) with the associated second coding means (25) is only insertable into the nebulizer (1) or usable therewith when the coding means (24, 25) have a matching coding, wherein at least one of the coding means (24, 25) comprises at least one, particularly web-like, nose-like or thread-like, projection (30) projecting transversally to the direction of insertion (E) of the container (3), and wherein at least one of the coding means (24, 25) comprises at least one preferably groove-like or thread-like recess (31) formed transversally to the direction of insertion (E) of the container (3),
**characterised**
**in that** the projection (30) and the recess (31) are constructed and cooperate such that, when the coding matches, during insertion of the container (3) into the nebulizer (1) they can be brought into engagement and axially pushed into each other.

2. Nebulizer according to claim 1, **characterised in that** the coding means (24, 25) are constructed so that, when the coding does not match, at least full insertion of the container (3) is prevented, in particular the establishing of contact of the fluid (2) with the pressure generator (5) or a conveying tube (9) being prevented, and/or **in that** the coding means (24, 25) are constructed so that when the coding does not match they prevent the housing part (18) from being mounted on the nebulizer (1).

3. Nebulizer according to any one of the preceding claims, **characterised in that** the coding means (24, 25) cooperate, preferably exclusively, mechanically, and/or in that the coding means (24, 25) cooperate electrically, inductively, capacitively, magnetically and/or optically, and/or **in that** the first coding means (24) is mounted or mountable on the nebulizer (1) non-detachably.

4. Nebulizer according to any one of the preceding claims, **characterised in that** the first coding means (24) is mounted on the nebulizer (1) in a force-fit and/or form-fit manner, in particular is clipped, clamped, stuck, injection moulded, screwed and/or formed thereon and/or integrated therein, and/or **in that** the first coding means (24) is arranged on or in the region of an insertion opening (27) of the nebulizer (1) for the container (3), and/or **in that** the first coding means (24) is arranged on or in the region of a holder (6) of the nebulizer (1) for the container (3).

5. Nebulizer according to any one of the preceding claims, **characterised in that** the second coding means (25) corresponds to the fluid (2) in the container (3), a concentration of active substance in the fluid (2) and/or an amount of fluid.

6. Nebulizer according to any one of the preceding claims, **characterised in that** the second coding means (25) is, preferably non-detachably, mounted or mountable on the container (3), in particular wherein the second coding means (25) is mounted on the container (3) in a force-fit and/or form-fit manner, particularly is clipped, clamped, stuck, injection moulded, screwed and/or formed thereon and/or integrated therein.

7. Nebulizer according to any one of the preceding claims, **characterised in that** the second coding means (25) is arranged on a head (28) of the container (3), in particular wherein the container (3) comprises, adjacent to the head (28), a radial indentation (29) in which the second coding means (25) engages for fixing to the container (3), and/or **in that** the second coding means (25) is mounted on a preferably cylindrical casing (33) of the container (3).

8. Nebulizer according to any one of the preceding claims, **characterised in that** the second coding means (25) is arranged on a base (21) of the container (3), in particular wherein the container (3) has a preferably radially widened end portion (34) which is encompassed by the second coding means (25) for fixing to the container (3).

9. Nebulizer according to any one of claims 1 to 5, **characterised in that** the second coding means (25) is arranged on, non-detachably mounted on or formed by the housing part (18).

10. Nebulizer according to claim 9, **characterised in that** the second coding means (25) comprises a radially inwardly open recess (31) extending axially and preferably formed on the inside of the outer wall of the housing part (18).

11. Nebulizer according to claim 9 or 10, **characterised in that** the first coding means (24) is constructed as a plug-in portion (32) which is mountable in latching and/or non-detachable manner on the inner part (17).

12. Nebulizer according to any one of the preceding claims, **characterised in that** the container (3) is non-detachably connected to the housing part (18).

13. Nebulizer according to any one of the preceding claims, **characterised in that** at least one of the coding means (24, 25) is formed ring-shaped, sleeve-shaped, bracket-like, cam-like, web-like, groove-like and/or hook-like.

14. Nebulizer according to any one of the preceding claims, **characterised in that** the coding means (24, 25) can be brought into engagement with one another by a linear and/or screwing movement when the coding matches, and/or **in that** the coding means (24, 25) can move past one another during insertion of the container (3) when the coding matches or engage with one another once the container (3) has been inserted or the coding of the first and/or second coding means (24, 25) is, preferably retrofittingly, adjustable and/or definitively fixable.

15. Nebulizer according to any one of the preceding claims, **characterised in that** the coding means (24, 25), particularly projections (30) and/or recesses (31) of the coding means (24, 25), correspond in their angular arrangement with regard to the direction of insertion (E) of the container (3) only when the coding matches, and/or in that the coding means (24, 25), in particular projections (30) and/or recesses (31) of the coding means (24, 25), correspond in their longitudinal extent in the direction of insertion (E) of the container (3) only when the coding matches, and/or **in that** the first coding means (24) and the second coding means (25) are designed complementary to each other when the coding matches.

16. Nebulizer according to any one of the preceding claims, **characterised in that** the container (3) is rotationally symmetrical and/or rigidly constructed, and/or **in that** the pressure generator (5) can be tensioned counter to spring force, and/or **in that** the pressure generation and/or nebulization is carried out purely mechanically, particularly without propellant gas, preferably by spring force, and/or **in that** the nebulizer (1) is constructed as an inhaler, particularly for medicinal aerosol treatment.

17. Use of a container (3) with a nebulizer (1), wherein the nebulizer (1) comprises a pressure generator (5) for conveying and/or nebulizing the fluid (2) and preferably a housing part (18) which is detachable for the insertion of the container (3) and a first coding means (24), wherein the container (3) is associated with a second coding means (25), the coding means (24, 25) cooperating with one another such that the container (3) with the associated second coding means (25) is only insertable in the nebulizer (1) when the coding means (24, 25) have a matching coding,
**characterised**
**in that** the coding is first adjusted and then finally fixed, wherein the coding means (24, 25) are designed in such a way that at least the coding of the first coding means (24) is compulsorily set only with insertion of the container (3) in the nebulizer (1) for the first time.

## Revendications

1. Atomiseur (1) pour un fluide (2), avec un récipient (3) insérable, contenant le fluide (2), avec un générateur de pression (5) fonctionnant seulement mécaniquement et actionnable manuellement pour le refoulement et/ou la vaporisation du fluide (2) et préférentiellement avec une pièce de boîtier (18) amovible pour l'insertion du récipient (3), l'atomiseur (1) comportant un premier moyen de codage (24) et un deuxième moyen de codage (25) étant associé au récipient (3), les moyens de codage (24, 25) coopérant de telle manière que le récipient (3) avec le deuxième moyen de codage (25) associé ne peut être mis en place dans l'atomiseur (1) ou n'être utilisable avec celui-ci que si les moyens de codage (24, 25) présentent des codages adaptés l'un à l'autre, au moins un des moyens de codage (24, 25) présentant au moins une saillie (30) transversale au sens d'insertion (E) du récipient (3), ayant en particulier une forme de nervure, d'ergot ou de filet, et au moins un des moyens de codage (24, 25) présentant au moins un évidement (31) ménagé transversalement au sens d'insertion (E) du récipient (3), ayant préférentiellement une forme de rainure ou de filet,
**caractérisé**
**en ce que** la saillie (30) et l'évidement (31) sont réalisés et coopèrent de manière à pouvoir être mis en prise et être poussés axialement l'une dans l'autre en cas de codages adaptés lors de l'insertion du récipient (3) dans l'atomiseur (1).

2. Atomiseur selon la revendication 1, **caractérisé en ce que** les moyens de codage (24, 25) sont réalisés de manière à empêcher au moins l'insertion complète du récipient (3), en particulier la réalisation d'un contact du fluide (2) avec le générateur de pression (5) ou un conduit de refoulement (9) en cas de codages inadaptés, et/ou en ce que les moyens de codage (24, 25) sont réalisés de manière à empêcher l'application de la pièce de boîtier (18) contre l'atomiseur (1) en cas de codages inadaptés.

3. Atomiseur selon l'une des revendications précédentes, **caractérisé en ce que** les moyens de codage (24, 25), coopèrent mécaniquement, préférentiellement de manière exclusive, et/ou **en ce que** les moyens de codage (24, 25) coopèrent électriquement, inductivement, capacitivement, magnétiquement et/ou optiquement, et/ou **en ce que** le premier moyen de codage (24) est applicable ou appliqué de manière inamovible sur l'atomiseur (1).

4. Atomiseur selon l'une des revendications précédentes, **caractérisé en ce que** le premier moyen de codage (24) est appliqué par serrage et/ou par engagement positif sur l'atomiseur (1), en étant en particulier enclenché, pincé, collé, injecté, vissé et/ou moulé et/ou est monté dans celui-ci, et/ou **en ce que** le premier moyen de codage (24) est disposé contre ou dans la zone d'une ouverture d'insertion (27) de l'atomiseur (1) pour le récipient (3), et/ou **en ce que** le premier moyen de codage (24) est disposé contre ou dans la zone d'un support (6) de l'atomiseur (1) pour le récipient (3).

5. Atomiseur selon l'une des revendications précédentes, **caractérisé en ce que** le deuxième moyen de codage (25) correspond au fluide (2) dans le récipient (3), à une concentration de substance active du fluide (2) et/ou à une quantité de fluide.

6. Atomiseur selon l'une des revendications précédentes, **caractérisé en ce que** le deuxième moyen de codage (25) est applicable ou appliqué préférentiellement de manière inamovible sur le récipient (3), le deuxième moyen de codage (25) étant en particulier appliqué par serrage et/ou par engagement positif sur le récipient (3), en étant en particulier enclenché, pincé, collé, injecté, vissé et/ou moulé et/ou étant monté dans celui-ci.

7. Atomiseur selon l'une des revendications précédentes, **caractérisé en ce que** le deuxième moyen de codage (25) est disposé sur une tête (28) du récipient (3), le récipient (3) présentant en particulier une indentation radiale (29) adjacente à la tête (28), dans laquelle s'engage le deuxième moyen de codage (25) pour fixation sur le récipient (3), et/ou **en ce que** le deuxième moyen de codage (25) est appliqué contre un corps (33) préférentiellement cylindrique du récipient (3).

8. Atomiseur selon l'une des revendications précédentes, **caractérisé en ce que** le deuxième moyen de codage (25) est appliqué contre un fond (21) du récipient (3), le récipient (3) présentant en particulier une zone d'extrémité (34) de préférence à élargissement radial, laquelle entoure le deuxième moyen de codage (25) pour fixation sur le récipient (3).

9. Atomiseur selon l'une des revendications 1 à 5, **caractérisé en ce que** le deuxième moyen de codage (25) est disposé contre la pièce de boîtier (18), en étant appliqué de manière inamovible ou en étant formé par celle-ci.

10. Atomiseur selon la revendication 9, **caractérisé en ce que** le deuxième moyen de codage (25) présente un évidement (31) ouvert radialement vers l'intérieur, à extension axiale et préférentiellement formé sur la face intérieure de la paroi extérieure de la pièce de boîtier (18).

11. Atomiseur selon la revendication 9 ou 10, **caractérisé en ce que** le premier moyen de codage (24) est réalisé comme pièce enfichable (32), applicable par enclenchement et/ou de manière inamovible sur la pièce intérieure (17).

12. Atomiseur selon l'une des revendications précédentes, **caractérisé en ce que** le récipient (3) est raccordé de manière inamovible à la pièce de boîtier (18).

13. Atomiseur selon l'une des revendications précédentes, **caractérisé en ce qu'**au moins un des moyens de codage (24, 25) est réalisé avec une forme d'anneau, de manchon, d'agrafe, de came, de nervure, de rainure et/ou de crochet.

14. Atomiseur selon l'une des revendications précédentes, **caractérisé en ce que** les moyens de codage (24, 25) peuvent être mis en prise par mouvement linéaire et/ou vissage en cas de codage adapté, et/ou **en ce que** les moyens de codage (24, 25) sont déplaçables l'un devant l'autre lors de la mise en place du récipient (3) en cas de codage adapté, ou sont en prise quand le récipient (3) est mis en place, ou le codage du premier et/ou du deuxième moyen de codage (24, 25) est réglable de préférence ultérieurement et/ou peut être défini définitivement.

15. Atomiseur selon l'une des revendications précédentes, **caractérisé en ce que** les moyens de codage (24, 25), en particulier les saillies (30) et/ou les évidements (31) des moyens de codage (24, 25) ne correspondent par leur disposition angulaire par rapport au sens d'insertion (E) du récipient (3) qu'en cas de codage adapté, et/ou en ce que les moyens de codage (24, 25), en particulier les saillies (30) et/ou les évidements (31) des moyens de codage (24, 25) ne correspondent par leur extension longitudinale dans le sens d'insertion (E) du récipient (3) qu'en cas de codage adapté, et/ou **en ce que** le premier moyen de codage (24) et le deuxième moyen de codage (25) sont configurés de manière complémentaire l'un à l'autre en cas de codage adapté.

16. Atomiseur selon l'une des revendications précédentes, **caractérisé en ce que** le récipient (3) est prévu à symétrie de rotation et/ou rigide, et/ou **en ce que** le générateur de pression (5) en pouvant être serré contre la force d'un ressort, et/ou **en ce que** la génération de pression ou la vaporisation sont effectuées de manière purement mécanique, en particulier sans gaz propulseur, préférentiellement par la force d'un ressort, et/ou **en ce que** l'atomiseur (1) est réalisé comme inhalateur, en particulier pour un traitement médical par aérosol.

17. Utilisation d'un récipient (3) avec un atomiseur (1), l'atomiseur (1) comportant un générateur de pression (5) pour le refoulement et/ou la pulvérisation d'un fluide (2) et préférentiellement une pièce de boîtier (18) amovible pour l'insertion du récipient (3) et un premier moyen de codage (24), un deuxième moyen de codage (25) étant associé au récipient (3) et les moyens de codage (24, 25) coopérant de telle manière que le récipient (3) avec le deuxième moyen de codage (25) associé ne peut être mis en place dans l'atomiseur (1) que si les moyens de codage (24, 25) présentent des codages adaptés l'un à l'autre,
**caractérisée**
**en ce que** le codage est réglé en premier lieu puis fixé de manière définitive, les moyens de codage (24, 25) étant conçus de telle sorte qu'au moins le codage du premier moyen de codage est obligatoirement réglé seulement lors de la première insertion du récipient (3) dans l'atomiseur (1).
